# EUROPEAN PATENT SPECIFICATION

(11) **EP 4 554 453 B1**
(45) Date of publication and mention of the grant of the patent: **15.04.2026**
(21) Application number: 23748289.8
(22) Date of filing: 12.07.2023
(51) Int. Cl.: A61B 5/00, A61B 5/291

(54) **AN IMPLANTABLE SENSOR AND A WEARABLE DETECTOR**
IMPLANTIERBARER SENSOR UND TRAGBARER DETEKTOR
CAPTEUR IMPLANTABLE ET DÉTECTEUR POUVANT ÊTRE PORTÉ

(30) Priority: 12.07.2022 GB 202210219
(43) Date of publication of application: 21.05.2025
(73) Proprietor: Coherence Neuro Ltd, Chesterton, Cambridge CB4 1NQ (GB)
(72) Inventor: WOODINGTON, Ben, London EC2A 2BB (GB); JENKINS, Elise, London EC2A 2BB (GB); MIDDYA, Sagnik, London EC2A 2BB (GB); BRAENDLEIN, Marcel, London EC2A 2BB (GB)
(74) Representative: Appleyard Lees IP LLP
(86) International application number: PCT/GB2023/051824
(87) International publication number: WO 2024/013493

(56) References cited:
- US-A1- 2014 072 308
- US-A1- 2020 046 224
- US-A1- 2021 133 528
- US-A1- 2021 313 529
- US-B1- 9 880 148
- ALI NAWAZ ET AL: "Organic Electrochemical Transistors for In Vivo Bioelectronics", ADVANCED MATERIALS, VCH PUBLISHERS, DE, vol. 33, no. 49, 4 October 2021 (2021-10-04), pages n/a, XP072497991, ISSN: 0935-9648, DOI: 10.1002/ADMA.202101874

## Description

### Field

The present techniques generally relate to implantable sensors. In particular, the present techniques provide an implantable sensor for detecting physiological signals within a patient, and a wearable detector for receiving sensed data from the implantable sensor.

### Background

Measuring physiological signals using smart devices has become increasingly popular. Usually, these measurements are limited to signals, such as heart rate, that can be detected from outside the body, and are associated with relatively few data points. However, with improvements in computing, physiological signals of ever-increasing complexity can be processed and analysed.

In particular, physiological signals measured from inside the body can provide information at a level of detail that cannot be attained by measurements made outside the body. For example, sensors inside the body may be able to monitor a single nerve or provide information about brain chemistry. However, being able to measure signals from inside the body in a way that enables real-time, accurate and unobtrusive measurements is a challenge. Current methods used to transmit physiological signals involve either a physical connection (i.e. electron transfer), or in the case of a wireless signal, signal transfer using ultrasound and/or radio frequencies.

Hence, current methods present obvious disadvantages. A physical connection is invasive and has to remain in place during use, making it impractical for real-time applications. Transmission using ultrasound frequencies, on the other hand, poses a risk of tissue heating and other potential biological responses, especially when used to transmit physiological signals from inside the brain.

A device capable of real-time data transmission, without the above mentioned disadvantages has numerous applications, for example, in monitoring brain activity and detecting epileptic seizures or other events, and alerting a user early to the occurrence of such an event. Additionally, any device for monitoring signals from inside the body would have to be easy to implant and resistant to any rejection reactions, as well as be able to provide accurate real-time data.

Background information can be found in: US9880148 which discloses a graphene-based neuroelectric sensor and stimulator system; US2014/072308 which discloses an implantable bio-sensing platform architecture; and US2020/0046224 which discloses implantable tunable medical detectors. All of these documents describe sensors and detectors that have some of the disadvantages described above or are simply not suitable for implant.

Further background information can be found in US20210313529A1., which discloses an internal ion-gated organic electrochemical transistor (IGT) having a channel formed of a material that includes a conducting polymer and a biocompatible solution.

Further background information can be found in US2021/0133528 which discloses untethered devices for deployment in living objects.

The applicant has therefore identified the need for an improved sensor for monitoring physiological signals.

### Summary

In a first approach of the present techniques, there is provided an implantable sensor for measuring a physiological signal, the sensor comprising: at least one transistor for sensing the physiological signal; at least one light source, coupled to the transistor, for transmitting data to an external detector, wherein the at least one light source emits light of a predetermined intensity and frequency in response to the sensed physiological signal; at least one power source to power the at least one transistor and the at least one light source; and a magnetic component (108) for orienting the implantable sensor (100) within a body, such that light emitted by the at least one light source (104) is detectable by an external detector.

Thus, the present techniques provide an implantable sensor, which may be provided within a human or animal body, which advantageously senses a physiological signal and wirelessly transmits data to an external detector in response to the sensed physiological signal. The present sensor is therefore advantageous over large, bulky, hard-wired communication techniques often used in typical implantable medical devices. The implantable sensor can be made small enough so that it is injectable into a patient using a fine needle rather than requiring extensive surgery. For example, the implantable sensor may have a width <0.5mm, a length <2mm, and a maximum thickness of 300µm. It will be understood that these are example, non-limiting dimensions that may make the sensor suitable to be injected into a patient.

There are a number of reasons that the present techniques provide a sensor which is small enough to be injectable/implantable.

Firstly, using the at least one transistor to sense the physiological signal means that the measured physiological signal is actively amplified, i.e. the sensor is an active sensor. By comparison, using an electrode pair to sense a physiological signal requires an amplifying circuit to amplify the signal, and consequently logic/control and secondary power components to control and power the amplifying circuit. That is, using a transistor to measure the physiological signal significantly decreases the number of components that the sensor comprises and thus enables miniaturisation.

Secondly, as mentioned above, the light source(s) emit light of a predetermined intensity and frequency in response to the sensed physiological signal. Thus, the at least one light source's output is effectively controlled by the transistor. This means that the implantable sensor can function with three key components only: at least one power source, at least one transistor and at least one light source. Each of these components can have very small dimensions, for example, the transistor can have dimensions on the order of 10s of µ*m* and the light source can have dimensions on the order of <200 *µm*. The largest component in the present sensor may be the power source. The present applicant has cleverly designed the present sensor in such a way that the number of active components required for the sensor to function has been greatly reduced. This enables the sensor to be significantly smaller than any existing sensors. In turn, this miniaturisation, is key for reducing a surgical window required to implant the sensor, substantially reducing risks and offering/allowing for wider clinical application/adoption.

As noted above, the power source may be the largest of the three key components of the sensor. In some cases, the power source of the implantable sensor may be a wireless power receiver, which further reduces the size of the sensor. This is because a wireless power source may have dimensions that are significantly smaller than those required by other power sources, such as batteries.

The sensor may comprise a single transistor and a single light source coupled to the transistor. Alternatively, the sensor may comprise a plurality of transistors coupled to/multiplexed to a single light source. Alternatively, the sensor may comprise a plurality of transistors and a plurality of light sources, where each transistor may be coupled to one or more light sources.

The at least one transistor for sensing the physiological signal may be an organic electrochemical transistor, OECT. Additionally, or alternatively, the at least one transistor may be an internally gated transistor (IGT). An internally gated transistor, as referred to herein, means an internal ion-gated organic electrochemical transistor.

The at least one power source may be any of: a battery, a supercapacitor, a conductive polymer supercapacitor, an electromagnetic wireless power receiver, an acoustic power receiver, an optical power receiver, a wireless power receiver, and an energy harvesting device. Broadly speaking, the power source may be one of two types: a power receiver or an energy store (e.g. a battery or supercapacitor). In some cases, the power source may comprise both a power receiver and an energy store. The power receiver may, for example, charge the battery as well as power the other components of the sensor. In this way, the battery may be used to power components of the sensor when the power receiver is not receiving power.

The sensor may comprise a polymer substrate. The polymer substrate may be formed of a flexible material. The polymer substrate may be formed of an electrically insulating material.

In some cases, components of the sensor may be distributed on both sides or surfaces of the polymer substrate. In a first example, source, drain and gate electrodes and a channel of the at least one transistor may be provided on a first surface of the polymer substrate. The light source and power source may be provided on a second surface of the polymer substrate. In this way, the components are distributed on the first and second surfaces of the substrate.

In a second example, a channel of the at least one transistor may be provided on a first surface of the polymer substrate, and source, drain and gate electrodes of the at least one transistor may be provided on a second surface of the polymer substrate.

When the sensor comprises a plurality of transistors, the transistors may all be on the same surface/side of the substrate, or may be distributed across both surfaces/sides. In both cases, the transistors may be formed as per the first or second examples above. In a particular example, the channels of the transistors may be provided on both the first and second surfaces, while the source, drain and gate electrodes may be provided on one surface of the polymer substrate only. This may be advantageous as it may improve the efficiency and speed to manufacture the sensor.

In some cases, the light source and power source may be provided on the second surface of the polymer substrate.

For the or each transistor, the source and drain electrodes are insulated, and the channel is exposed. Insulated means that an electrode or other part of the sensor is insulated from the sensor's surroundings, meaning that an insulated electrode is not in contact with any ions or other physiology at the sensor's implantation site. For the or each transistor, the source drain and gate electrodes may be metallic. For example, the source, drain and gate electrodes may be made from gold (Au) or platinum (Pt). In some cases, the gate electrode may be functionalised. A functionalised gate electrode is a gate electrode that is coated in a material suitable for sensing a chemical signal, such as a specific biological species or specific physiological signal (e.g. neurotransmitters, ions etc.). This may be useful when the sensor is used to sense chemical/other physiological signals, such as specific ions and/or neurotransmitters. The source, drain and gate electrodes may be insulated using a polymer material. Alternatively, the gate electrode may also be exposed. This may be useful when the sensor is used to measure electrical signals (i.e. electrophysiological signals). In this case, the gate electrode may be used to obtain a reference signal for the body, and the channel's doping/de-doping may be with respect to the body's reference signal.

For the or each transistor, the channel (and the gate electrode, if also exposed) may be formed of a material suitable for sensing the physiological signal. The channel may be formed of a conductive polymer material such as, but not limited to, poly(3,4-ethylenedioxythiophene) polystyrene sulfonate (PEDOT:PSS).

The light source and power source may be coupled to the source and drain electrodes of the at least one transistor.

In some cases, the substrate may be substantially planar.

In other cases, the substrate may comprise a first portion and a second portion, and the first portion may be at an (non-zero) angle relative to the second portion. In such cases, the at least one light source may be provided on the first portion and the at least transistor may be on the second portion. For example, the first portion and second portion of the substrate may form an 'L' shape or a 'T' shape. This non-planar substrate may be advantageous when the sensor is implanted into the brain or other tissue, because the at least one transistor may be located deeper within the tissue than the light source. This may enable the light source to transmit data to an external detector more easily than if the light source were located at the same depth as the transistor(s). That is, using a non-planar substrate enables a closer alignment between the light source and the external detector in terms of an incidence angle of the light emitted by the light source that is then detected by the external detector. That is, a more favourable incidence angle of light emitted by the light source results in the light travelling a shorter path to reach the detector, meaning that a signal of higher intensity reaches the detector. Additionally, a more favourable incidence angle may mean that the detector is placed relative to the sensor in such a way that a substantial portion of the light emitted by the light source of the sensor is incident on the detector. Optimising an incidence angle may mean that the first portion may be at any angle between 90° (a 'bent' sensor) and 180° (a 'flat' or planar sensor) relative to a second portion. An angle between the first and second portions may be achieved by "bending" the sensor before implantation, or the sensor may be flexible enough that such "bending" happens simply by placing the sensor into the brain or other physiological structure. It will be understood that there may be other ways to form the non-planar substrate and that bending is a non-limiting example.

The light source may be a light-emitting diode (LED) and/or an organic light-emitting diode (OLED), such as a near-infrared LED or a visible light LED. The light source may be an organic light-emitting diode (OLED), such as a near-infrared OLED or a visible light OLED. The light source may also be a laser diode. The choice of light source, and/or wavelength and/or frequency emitted by the light source, may depend on the sensor's location in the body. For example, when the sensor is located close to the surface of the body, visible light may be used. In another example, when the light source is located deeper within a body such that there is more tissue through which the light emitted by the light source has to travel before reaching the external detector, the wavelength of the light emitted by the light source may be increased and/or the intensity of the light emitted by the light source may be increased.

The sensor may be partially encapsulated in an optically-transparent material to protect components of the sensor from moisture ingress and damage. For example, the at least one light source of the sensor may be encapsulated. In another example, the whole sensor may be encapsulated except for the channel of the transistor(s) which is exposed. The optically-transparent material used for the encapsulation may be a polymer and/or a metal oxide. The polymer may, for example, be Parylene-C (PaC) or any suitable transparent polymer (e.g. polyamide). In some cases, the sensor may be (partially) encapsulated by a plurality of layers of the optically-transparent material.

Alternatively, the sensor may be encapsulated in a protective material. For example, the protective material may be glass, rubber, silicon, ruby, ceramic, and so on. The protective material preferably comprises one or more windows/apertures or cut-outs to enable certain components of the sensor to function. For example, the protective material may comprise a window over the at least one light source so that the light source is able to transmit data to an external detector. Similarly, the protective material may comprise a window over the channel of the transistor(s), and the power source where this is a wireless power source. The window is preferably formed of a transparent material such as glass or ruby.

The sensor may further comprise a component for delivering electrical stimulation. The component may be or comprise at least one electrode for delivering electrical stimulation. This may enable the sensor to, for example, stimulate brain activity or otherwise apply energy to the user's body, and resulting brain activity or physiological signals may be measured by the at least one transistor. The component may be driven by an actuator.

The at least one electrode may be coupled to the power source. That is, the at least one electrode may be powered by the power source.

The sensor comprises a magnetic component for orienting the implantable sensor within a body, such that light emitted by the light source is detectable by an external detector.

The magnetic component may be adjacent to the light source.

The sensor may further comprise a modulator for modulating light emitted by the light source in response to the sensed physiological signal. Where there are multiple light sources, there may be a single or multiple modulators.

The modulator may modulate any one of the following properties of the emitted light: pulse, amplitude, frequency, phase and intensity.

When no physiological signal is sensed by the transistor, the modulator may modulate the light emitted by the light source so that the light is emitted at a first frequency and a first intensity, and when the transistor senses the physiological signal, the modulate may modulate the light emitted by the light source so that the light is emitted at a second frequency and a second intensity.

The physiological signal sensed by the sensor may be a neural signal, for example, a signal resulting from an electrophysiological epoch or a specific chemical signal, for example, a signal from a specific ion and/or a specific neurotransmitter. The physiological signal may be any one of: a chemical signal or substance, a biological signal or substance, or an electrical signal.

In a second approach of the present techniques, there is provided a wearable detector for receiving light signals transmitted by an implantable sensor as described herein, the detector comprising: at least one photodetector for detecting light signals transmitted by the implantable electronic sensor; a processor for processing the detected light signals; a magnet (210) for magnetically coupling to the magnetic component of the implantable sensor (100) and orienting the implantable sensor (100) inside the body; and a communication module configured to transmit the detected light signals to an external computing device.

The photodetector may be a near-infrared or visible light photodetector.

The detector may further comprise a wireless power transfer module configured to wirelessly transfer power to the implantable sensor.

The communication module may transmit the detected light signals to an external computing device via a wired or wireless communication means.

In a third approach of the present techniques, there is provided a system for measuring physiological signals, the system comprising: at least one implantable sensor, the sensor comprising: at least one transistor for sensing the physiological signal, at least one light source, coupled to the at least one transistor, for transmitting data to a detector, wherein the at least one light source emits light of a predetermined intensity and frequency in response to the sensed physiological signal, and a power source to power the at least one transistor and the at least one light source; and at least one wearable detector for receiving light signals transmitted by the at least one implantable sensor, the detector comprising: at least one photodetector for detecting light signals transmitted by the implantable electronic sensor, a processor for processing the detected light signals, and a communication module configured to transmit the detected light signals to an external computing device.

The features described above with respect to the first and second approaches apply equally to the third approach.

In a fourth approach of the present techniques, there is provided a method of manufacturing the implantable sensor described herein, the method comprising: providing a polymer substrate; forming source, drain and gate electrodes of the at least one transistor on the polymer substrate; and providing the light source and the power source on the polymer substrate.

In one example, the source, drain and gate electrodes and the channel of the at least one transistor may be provided on a first surface of the polymer substrate, and the light source and the power source may be provided on a second surface of the polymer substrate.

In another example, the channel of the at least one transistor may be provided on a first surface of the polymer substrate, and the source, drain and gate electrodes of the at least one transistor, the light source and the power source may be provided on a second surface of the polymer substrate.

In another example, the sensor may comprise a plurality of transistors. In this case, a channel of at least one transistor may be provided on a first surface of the polymer substrate, and a channel of at least one other transistor may be provided on a second surface of the polymer substrate.

The substrate may comprise a first portion and a second portion and the method may comprise: forming the substrate so the first portion is at an angle relative to the second portion; and providing the light source on the first portion and the at least one transistor on the second portion.

The method may further comprise partially encapsulating the sensor in an optically-transparent material.

The polymer substrate may be formed of an electrically insulating material. The polymer substrate may be flexible. The channel may be formed of a conductive organic polymer.

### Brief description of the drawings

Implementations of the present techniques will now be described, by way of example only, with reference to the accompanying drawings, in which:
Figure 1 is a schematic block diagram of a system for measuring physiological signals;
Figure 2 is a circuit diagram of the implantable sensor;
Figure 3 is a schematic diagram of a process to manufacture the implantable sensor;
Figures 4A to 4C show diagrams of layers of the implantable sensor having a single light source;
Figures 4D to 4F show diagrams of layers of the implantable sensor having multiple light sources and multiple transistors;
Figure 5 is a block diagram showing example processes performed by the system of Figure 1;
Figures 6A and 6B are schematic diagrams of an alternative process to manufacture the implantable sensor;
Figures 7A to 7D show example forms of the sensor; and
Figures 8A and 8B are block diagrams showing where example components of the system of Figure 1 may be provided on a user/patient.

### Detailed description of the drawings

Broadly speaking, embodiments of the present techniques provide an implantable sensor for detecting physiological signals within a patient, and a wearable detector for receiving sensed data from the implantable sensor. The implantable sensor comprises at least one transistor for sensing the physiological signal, at least one light source to emit light of a predetermined intensity and frequency in response to the sensed physiological signal, and a powering mechanism to power the at least one transistor and the at least one light source. Advantageously, the implantable sensor can be made small enough so that it is injectable into a patient using a fine needle rather than requiring extensive surgery.

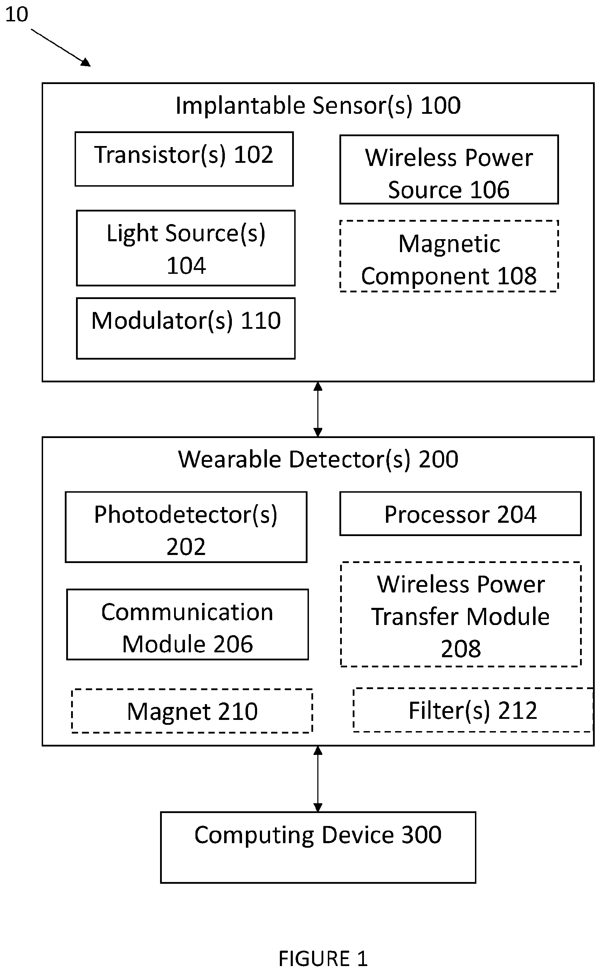

Figure 1 is a schematic block diagram of a system 10 for measuring physiological signals. The system may measure brain signals or other physiological signals within a human or animal body. For example, the system may be used to measure signals in the Central Nervous System (CNS) and/or the Peripheral Nervous System (PNS). The system 10 may be used for general computer-brain interfacing, or to detect or monitor certain diseases or conditions such as stroke, oncology, epilepsy, dementia and other neurological disorders. It will be understood this is an example list of disorders, diseases or conditions that could be detected or monitored using system 10. The system 10 may also be used to provide a peripheral neural interface and muscular interface, with applications in bionics.

The system 10 comprises at least one implantable sensor 100. In some cases, a single implantable sensor 100 may be implanted into a body in a specific location to understand the microenvironment of that location. For example, the implantable sensor 100 may be implanted into the central nervous system, on a peripheral nerve, or on/in a muscle. In another case, a plurality of implantable sensors 100 may be implanted into a region of the body using a 'scatter-shot approach', where the sensors are dispersed into a region rather than being implanted into a specific location. For example, multiple sensors 100 may be implanted into an organ, such as the brain. In either case, the sensors may be introduced into a body using a fine needle injection or via other minimally-invasive surgical techniques.

The sensor 100 comprises at least one transistor 102 for sensing the physiological signal. The or each transistor 102 is able to sense electrical signals produced by the body via the channel or gate electrode of the transistor. Specifically, the transistor(s) 102 may be able to sense potential differences. The transistor(s) 102 may also be functionalised sensor so that it can detect a specific signal or species/substance instead of potential differences. For example, the transistor(s) may be functionalised to detect a chemical signal or substance, a biological signal or substance, or an electrical signal (such as, for example, an electrocardiogram signal or electromyography signal). In some cases, the transistor(s) 102 may be an organic electrochemical transistor, OECT. In some cases, the transistor 102 may be an internally gated transistor (IGT). An internally gated transistor, as referred to herein, means an internal ion-gated organic electrochemical transistor.

The sensor 100 comprises at least one light source 104, coupled to the at least one transistor 102, for transmitting data to a detector. The at least one light source 104 emits light of a predetermined intensity and frequency in response to the sensed physiological signal.

The sensor 100 may comprise a single transistor 102 and a single light source 104 coupled to the transistor 102. Alternatively, the sensor 100 may comprise a plurality of transistors 102 coupled to/multiplexed to a single light source 104. Alternatively, the sensor may comprise a plurality of transistors 102 and a plurality of light sources 104, where each transistor may be coupled to one or more light sources.

In some cases, the light source(s) 104 may be a light-emitting diode (LED) or an organic light emitting diode (OLED). The light source may also be a laser diode. The light source(s) 104 may be near infrared or visible light LEDs. The light source(s) 104 may be any type of LED, for example, a conventional LED made from gallium arsenide (GaAs), gallium phosphide (GaP) or gallium arsenide phosphide (GaAsP). The light source(s) may also be any type of quantum dot, for example, doped zinc sulphide, lead sulphide, cadmium selenide or indium phosphide. The light source(s) may also be any type of QLED. The insulation and/or passivation of the light source may made from, for example, any epoxy material, silicone-derived material, parylene (C/N/D), any silicon oxide (SiOx), any silicon oxinitrite (SiOxNy), or silicon carbide (SiC). The light source(s) 104 may emit light at an intensity and frequency dependent on the signal detected by the transistor. The choice of light source, and/or wavelength and/or frequency emitted by the light source, may depend on the sensor's location in the body. When the sensor is located close to the surface of the body, visible light may be used. When there is more tissue through which the light emitted by the light source has to travel before reaching the detector, the wavelength of the light emitted by the light source may be increased and/or the intensity of the light emitted by the light source may be increased. Depending on the material choice for the channel of the transistor, the light source may be on or off when a signal is detected. The external detector 200 is then simply calibrated accordingly. Where there are multiple light sources 104, they may be of the same type or different, and may emit light having the same properties or one or more different properties.

Light emitted by the light source(s) 104 will be transmitted through the biologically relevant tissue to one or more light sensors located inside or outside of the body, which may be part of the detector 200. That is, a light sensor of the detector 200 may be located in the body in which the sensor is located, or may be located outside of the body. The NIR-LED may operate at wavelengths between 700 nm and 1700nm (NIR I-II). For manufacturing, a conductive bonding adhesive may be blade coated on the exposed terminals such that the NIR-LED can be suitably placed. When the NIR-LED contacts are top/bottom rather than bottom-bottom, the NIR-LED may be wire bonded to the secondary exposed terminal. Alternatively, an O/LED can be directly assembled on the flexible substrate.

The sensor 100 comprises at least one power source 106 to power the transistor 102 and the light source 104 (and any other components of the sensor that require power). Broadly speaking, the power source may be one of two types: a power receiver or an energy store (e.g. a battery or supercapacitor). In some cases, the power source may comprise both a power receiver and an energy store. The power receiver may, for example, charge the battery as well as power the other components of the sensor. In this way, the battery may be used to power components of the sensor when the power receiver is not receiving power.

The power source 106 may be any one of: a battery, a supercapacitor, a conductive polymer supercapacitor, an electromagnetic wireless power receiver, an acoustic power receiver, an optical power receiver, and an energy harvesting device.

The power source 106 may also be an energy converter that converts energy from radiofrequency, ultrasound or biological energy. Radiofrequency or ultrasound energy may be provided by the detector 200 which may emit radiofrequency or ultrasound energy. The emission of radiofrequency or ultrasound energy may be directed specifically at the implantable apparatus or it may be emitted in all directions. Radiofrequency or ultrasound energy may also be emitted in a predetermined range of directions, for example, aimed at a brain or other physiological structure. Additionally or alternatively, the power source 106 may be integrated into the light source 104. That is, the light source may also be the power source. For example, the light source may receive light and convert this into electrical energy to power the sensor, and may also output light/electromagnetic waves.

As explained in more detail below, the sensor 100 may comprise a polymer substrate. The polymer substrate may be formed of a flexible material. The polymer substrate may be formed of an electrically insulating material.

In some cases, components of the sensor may be distributed on both sides or surfaces of the polymer substrate. In a first example, source, drain and gate electrodes and a channel of the transistor are provided on a first surface of the polymer layer.

In a second example, a channel of the at least one transistor may be provided on a first surface of the polymer substrate, and source, drain and gate electrodes of the at least one transistor may be provided on a second surface of the polymer substrate.

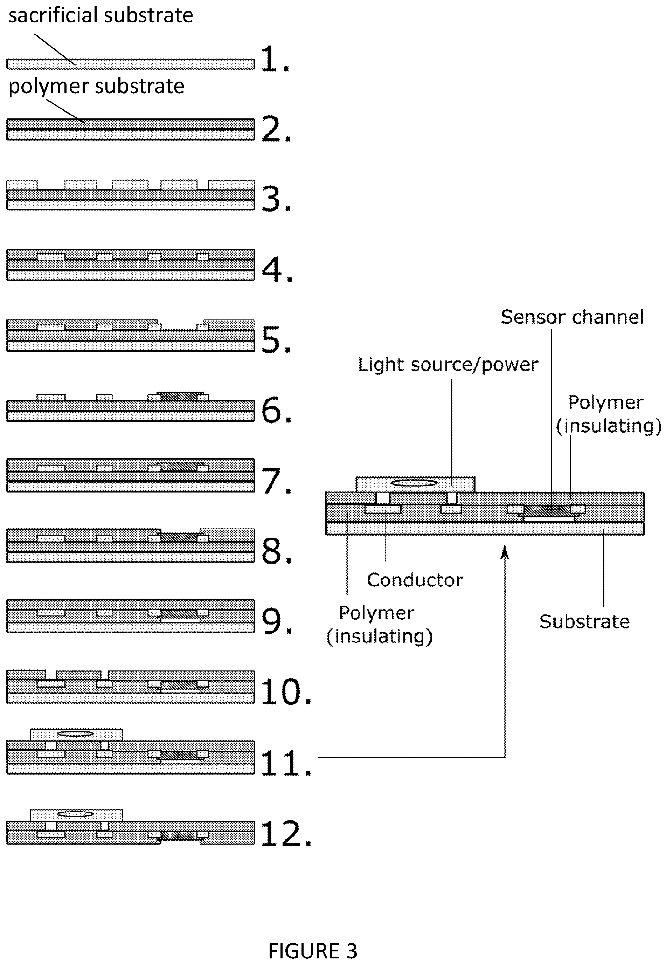

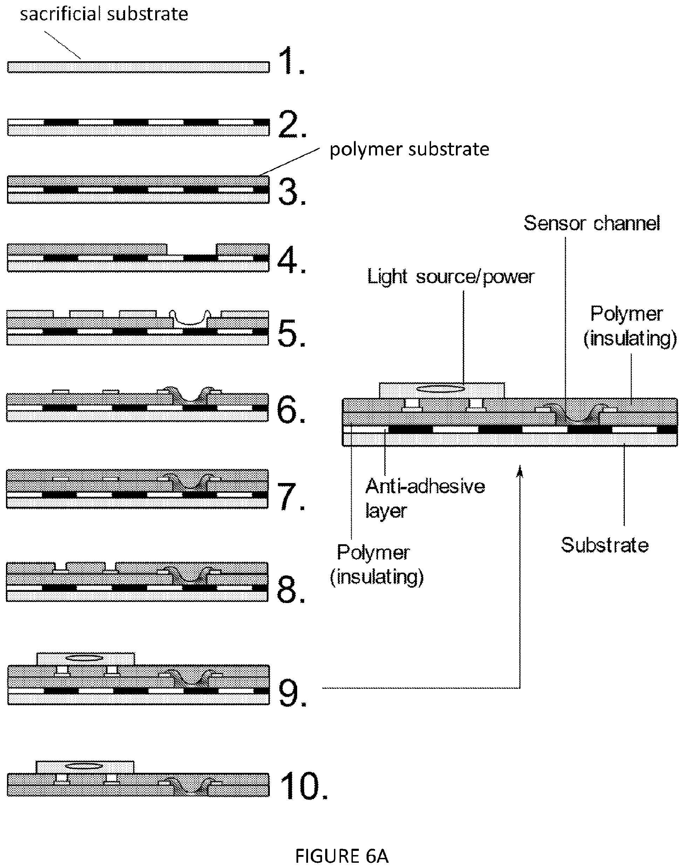

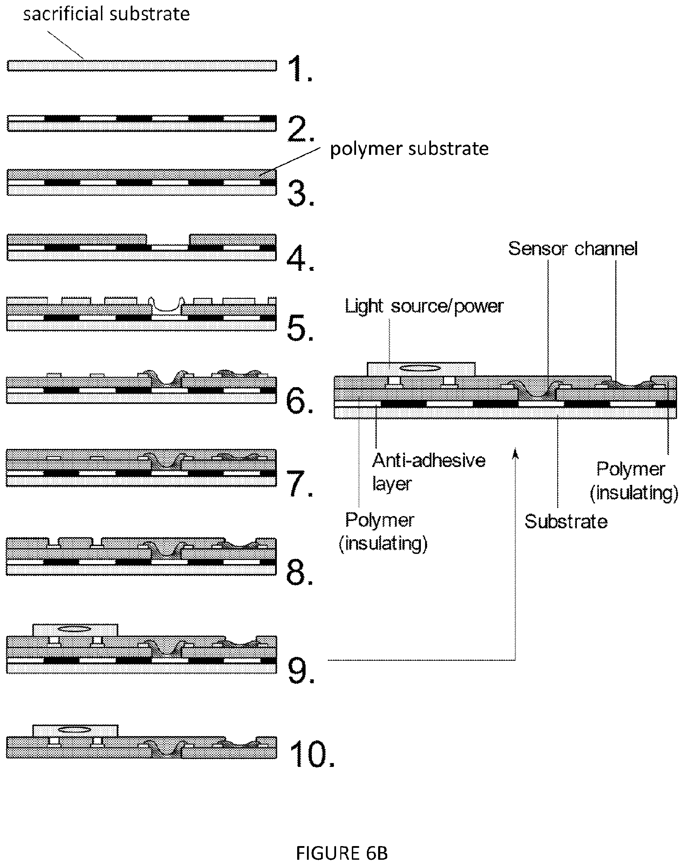

When the sensor comprises a plurality of transistors, the transistors may all be on the same surface/side of the substrate, or may be distributed across both surfaces/sides. In both cases, the transistors may be formed as per the first or second examples above. In a particular example, the channels of the transistors may be provided on both the first and second surfaces, while the source, drain and gate electrodes may be provided on one surface of the polymer substrate only. This may be advantageous as it may improve the efficiency and speed to manufacture the sensor. Example manufacturing processes are described in more detail with reference to Figures 3, 6A and 6B below.

For the or each transistor, the source and drain electrodes are insulated, and the channel is exposed. In some cases, the gate electrode may be functionalised or coated with a material that enables it to sense only a predetermined biological signal, such as a predetermined ion. That is, a functionalised gate electrode is a gate electrode that is coated in a material suitable for sensing specific biological species or specific physiological (e.g. neurotransmitters, ions etc.). This may be useful when the sensor is used to sense other physiological signals, such as specific ions and/or neurotransmitters. This could be useful when the sensor is used to sense chemical signals. The source, drain and gate electrodes may be insulated using a polymer material. Alternatively, the gate electrode may also be exposed. This may be useful when the sensor is used to measure non-chemical signals, i.e. the sum of any ionic changes happening. In this case, the gate electrode may be used to obtain a reference signal for the body, and the channel's doping/de-doping may be with respect to the body's reference signal.

For the or each transistor, the electrodes may be formed of any conductive material, such as a metal, metal alloy or a conductive polymer. The channel (and the gate electrode when also exposed) may be formed of a material suitable for sensing the physiological signal.

The light source and power source may be coupled to the source and drain electrodes of the/each transistor.

The polymer substrate, and any other insulation material of the sensor 100, may be made of any suitable material, preferably parylene-based polymers (C/N/D), polyimide, silicone-based polymers. The polymer substrate (also referred to herein as a substrate or base layer), and hence, the implantable sensor 100 may be flexible and conformable to biological tissue. This means that the implantable apparatus is less likely to be rejected by the body over time, and less likely to form tissue scarring which is also known as a foreign body reaction. For example, the implantable sensor 100 may be able to conform to the surface of the brain. The implantable sensor 100 may also be provided within the brain.

The transistor 102 may be produced using standard micro-fabrication and photolithographic techniques. The transistor may consist of a conducting contact source and drain insulated by an insulating material, such as Parylene-C (PaC) or any of those materials mentioned above, and separated by an exposed conducting channel. The conducting track leading from the drain-channel interconnect may be exposed for bonding with a light source 104, for example, a light emitting diode. The conducting track, as well as all electrical contacts may be made from any of gold, silver, platinum, palladium, iridium, iridium oxide and/or titanium, conductive polymers, or any other suitable material. The conducting track may be insulated/electrically separated from the sensing medium. The channel material determines the operating characteristics of the transistor because it determines whether the major charge carriers are holes or electrons. The transistor channel may be fabricated from a conductive polymer, such as PEDOT:PSS or another PEDOT derivate, which is a p-type material with a majority hole carrier. Used in the depletion mode, the material is de-doped by the electrical cations produced in the body due to, for example, a change in neural activity. These cations are injected into the channel (seen as a gate bias), where the de-doping restricts the electrical current to flow across the source to drain switching the device into an "off" state. Hence, the transistor is designed to operate with highest transconductance (gain) at essentially 0V gate bias. The frequency of the drain current directly infers the frequency of the field potentials in the local environment in which the measurements are taken. This fact is used for the coupled light source 104 to transmit physiological signals for deconvolution (i.e. analysis) outside of the body. That is, the light source 104 will transmit signals which are detected by a photodetector, and then analysed to determine what they mean. The transistor is not limited to p-type materials or operation in depletion mode. Many materials could be incorporated to operate in accumulation mode, such that an increase in cations surrounding the transistor would result in increased drain current.

The use of organic electrochemical transistors (OECTs) substantially improves the signal-to-noise ratio due to the large transconductance at the device's operating point. Hence, this results in more sensitive and local detection and amplification of electrical signals which means there is less noise present. Using an internally gated transistor (IGT) which relies on an ion reservoir within a channel further improves switching time of the transistor. That is, while an OECT relies on the body's electrolytes for conduction in a channel which can lead to slower switching times, an internally gated transistor eliminates the need for body electrolytes as conducting ions. This means that mobile ions within the gated channel, which may be protected by a membrane, can create a self-de-doping process which in turn leads to faster switch times. Faster switching times can then be utilised to increase the bandwidth of a signal emitted by the sensor. Additionally, detecting and transmitting the signal at the source, enables real-time operation, with transmission only limited by the bandwidth of the sensing technology (typically more than 1ms time constants for an OECT, and much faster for an IGT with time constants on the order of 2µs). The transistor may be chosen such that the gain of the transistor is highest at the sensed voltage of interest, which is typically between 0-0.1V for brain signals. It will be understood that the voltage of interest may be higher or lower depending on which parts of the body the sensor is being used to sense. Additionally, the voltage of interest may be influenced by the materials and/or dimensions of the OECT/ IGT (source, drain, gate, channel and electrical tracks/contact pads)

The sensor may be partially encapsulated to protect the at least one light source against moisture or damage. At least any moisture sensitive components of the sensor may be encapsulated to protect these components. Moisture sensitive components of the sensor may, for example, include the at least one light source (also referred to herein as an optical communication interface, such as an LED). Additionally or alternatively, the whole sensor apart from the at least one sensing channel may be encapsulated.

The sensor as may further comprise at least one electrode for delivering electrical stimulation. This may enable the sensor to, for example, stimulate brain activity or otherwise apply energy to the user's body, and resulting brain activity or physiological signals may be measured by the at least one transistor. The at least one electrode may be coupled to the power source. That is, the at least one electrode may be powered by the power source.

The sensor 100 comprises a magnetic component 108 for orienting the implantable sensor within a body, such that light emitted by the light source is detectable by an external detector. That is, the magnetic component 108 can be used to orient the sensor to ensure that the light source 104 emits light out of the body rather than into the body, so that the light can be detected. The magnetic component 108 may also enable the sensor 100 to be located within the body, so that it can be removed. Thus, preferably, the magnetic component is adjacent to the light source, i.e. on the same surface of the polymer substrate.

The sensor 100 may further comprise at least one modulator 110 for modulating light emitted by the light source in response to the sensed physiological signal. The number of modulators 110 may depend on the number of light sources 104. The modulator 110 may modulate any one of the following properties of the emitted light: pulse, amplitude, frequency, phase and intensity. For example, when no physiological signal is sensed by the transistor 102, the light source 104 may emit light at a first frequency, and when the transistor 102 senses the physiological signal, the light source 104 may emit light at a second frequency. The change in frequency can be detected by an external detector and used to determine that a physiological signal is present or absent. That is, the modulator may be used to encode the physiological signal before it is transmitted. The encoded signal may then be decoded by a detector, which detects the emitted light signal and decodes the light signal. Additionally, any of the modulation techniques described herein may also be used to provide calibration of the sensor and/or detector. That is, the modulation techniques may be used to communicate a position or location of a sensor within a cluster of sensors.

Intensity modulation involves using the modulator 110 to modulates the intensity of the light emitted by the light source.

Pulse modulation may involve pulse amplitude modulation (PAM) or pulse position modulation (PPM). In PAM, the information is encoded in the amplitude of a pulse. In PPM, information is encoded in the position of a pulse (the information is transmitted by a single impulsion at one of all possible time-shift). PAM can be accomplished by a square wave generator, resistances, and a transistor.

Amplitude modulation may be achieved using On-Off Keying (OOK).

Frequency modulation may be achieved using an Orthogonal Frequency Division Multiplexer (OFDM). In OFDM, the data is divided and modulated by multiple narrowband subcarriers. The simplest way to implement a frequency modulation (FM) requires a sine generator, transistors, and capacitors.

Spread spectrum techniques may also be used (where emitted power is spread in a large spectral domain). Spread spectrum techniques must be coupled with a modulation, therefore the implementation strongly depends on the modulation technique.

To successfully implement an optical wireless communication technique, filters may be used to capture different light wavelengths. For instance, narrowband IR filters designed for specific wavelengths would enable focus on a signal from one emitter among others operating at a different wavelength. The filters may be provided in a wearable detector for receiving light signals transmitted by the at least one implantable sensor 100.

Phase-shift keying (PSK) is a digital modulation process which conveys data by changing (modulating) the phase of a constant frequency reference signal (the carrier wave). The modulation is accomplished by varying the sine and cosine inputs at a precise time.

To further couple each light source 104 to a respective photodetector, the modulated drain current can be coupled to a carrier signal oscillator with the detected drain signal acting as information bearing signal. This way the carrier signal will be set for each distributed implantable sensor 100 in the system 10 having a unique, addressable and identifiable light signal. A method for deduction of these unique signals may include frequency shift keying, which is a frequency modulation scheme in which digital information is transmitted through discrete frequency changes of a carrier signal.

The system 10 also comprises at least one wearable detector 200 for receiving light signals transmitted by the at least one implantable sensor 100. When the implantable sensor(s) is implanted in the brain, the wearable detector(s) 200 may be worn on the head.

The detector 200 comprises at least one photodetector 202 for detecting light signals transmitted by the implantable electronic sensor 100, a processor 204 for processing the detected light signals, and a communication module 206 configured to transmit the detected light signals to an external computing device 300. The communication module 206 may transmit the detected light signals to the external computing device 300 via a wired or wireless communication means.

The photodetector 202 may be a near-infrared photodetector.

The detector 200 may further comprise a wireless power transfer module 208 configured to wirelessly transfer power to the implantable sensor.

The detector 200 further comprises a magnet 210 for magnetically coupling to the magnetic component of the implantable sensor and orienting the implantable sensor inside the body. The magnet 210 may comprise a small strip of magnetic material. This ensures that the implantable sensor 100 faces towards the detector 200, such that the light emitted by the light source 104 points in the direction of one or more detector(s) 200. For example, when the implantable apparatus is implanted onto the brain, the magnet may be used to ensure that the implantable apparatus points towards the inside of the skull/skin, rather than into the brain.

The light sensor 202 may be a near infrared (NIR) photodetector (PD) which converts received photons to electrons inducing an electrical current in response to light intensity. Similarly, the frequency or rate of the detected light signals reflect the frequency of the detected biological signals emitted by the light source 104 of the implantable sensor 100. The light sensor 202 may be connected to an operational amplifier and filtering circuit. Thresholding and calibration may be used to identify when a minimum "true" signal is detected. The detected signals will be demodulated and acquired at a suitable speed relevant to the maximum frequency of the biological input (<10kHz). Therefore, to achieve Nyquist sampling and avoid aliasing, a sampling rate of at least 20kHz is desirable.

As mentioned above, the wearable detector 200 may comprise one or more filters 212 to capture different light wavelengths transmitted by the at least one implantable sensor 100. For instance, narrowband IR filters designed for specific wavelengths would enable focus on a signal from one emitter among others operating at a different wavelength. The filters may be provided in a wearable detector for receiving light signals transmitted by the at least one implantable sensor 100.

The system 10 comprises a computing device 300, such as a computer, smartphone, or similar device. The computing device 300 may receive and store the data detected by the wearable detector 200.

Data detected and transmitted by the detector 200 may be received by the computing device 300. The computing device 300 may be a personal computer, mobile phone, tablet or any other computing device, which comprises a communication module configured to receive the data transmitted by the detector. The sampled information may additionally or alternatively also be transmitted to a remote server for processing. After the computing device 300 receives the sampled information, the sampled information may then, for example, be displayed to a user, and/or a physician or researcher. The computing device 300 may analyse the sampled information before it is displayed to a user.

The at least one implantable sensor may be implanted using a 'scatter-shot' approach, i.e. the implantable apparatus does not need to be placed in a predefined location. To then detect a signal from the at least one implantable apparatus, an array of detectors 200 is positioned on the outside of the body. Using this approach, the implantable apparatuses are distributed, enabling them to track electrical signals from various different locations or parts of the body, for example, the central and/or peripheral nervous system and/or muscles.

For example, it may be desirable to monitor a specific or several specific regions of the brain. Different regions of the brain produce distinct electrophysiological signals hence precise knowing of the implanted location may not be necessary. Additionally, the implantable apparatus may not remain in any one location but move around, especially when it is implanted onto the brain, which is surrounded by cerebrospinal fluid. The implantable apparatus may then remain mobile while inside the cerebrospinal fluid. After a while, however, the implantable apparatus may settle onto a location at the brain's surface, which is facilitated by the flexible nature of the substrate. Any signal analysis must therefore be capable of deriving the desired information without knowledge of the implantable apparatus' location.

The signals from the detectors may be used to train a model to infer neural state.

Use of an array of implantable sensors on the brain may enable the detection and analysis of more complex patterns. For example, decoding brain activity corresponding to speech may be possible with a 10x10 array of implantable sensors.

However, if the sensor used is a sensor for detecting a specific bodily signal/substance (chemical, biological, electrical or otherwise), only one implantable sensor may be needed to acquire sufficient data.

The implantable sensor may also be placed in a specific location to understand the microenvironment which is intended to be monitored - this may, for example, be in the central nervous system, on/embedded within a peripheral nerve, or on/embedded within a muscle, or embedded within any other type of tissue. For example, the sensor may be implanted in the subcutaneous space, fatty tissue or any other organs.

When analysing the signals received from the implantable sensor, it may also be necessary to take into account any diseases or other abnormalities or physiological differences of a subject. For example, the analysis may be adapted for a person with epilepsy, a stroke or brain cancer. If the implantable apparatus is implanted onto a brain, the frequencies that are detected can be specific to the type of disease or event that is being observed. For example, for brain cancer, there are often a lot of unexpected and erratic frequencies that can be observed. It is then possible to perform a Fourier transform (or a Fast Fourier transform), which shows which frequencies are detected by the implantable apparatus. To detect all potentially relevant frequencies, the implantable sensor must be able to cover a large bandwidth. It is also possible to apply low or high pass filters to the received signal during processing, so only frequencies of interest, which may be determined by the type of event or region that is to be observed, are used for analysis. Alternatively or additionally, a bandpass filter may be applied at the hardware level by the choice of sensor and/or sensor material.

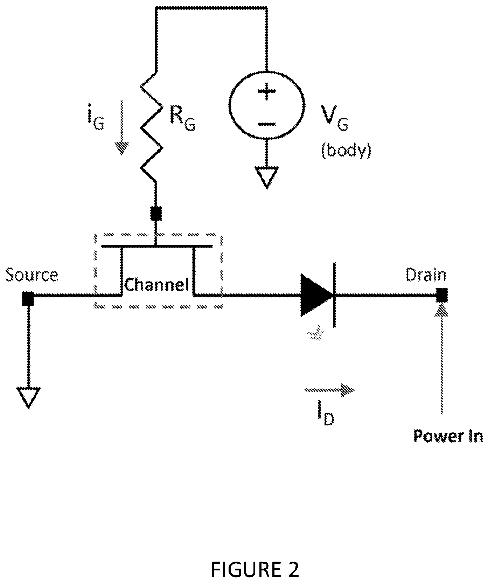

Figure 2 is a circuit diagram of the implantable sensor 100. I_{G} represents a gate current (transient), I_{D} represents a drain current, and R_{G} represents a gate resistance. V_{G} represents a gate signal provided by an electrical signal from the body (for example, cations injected into the channel) and requires a reference ground somewhere in or on the body or integrated into the implantable apparatus. Power is input into the sensor via the drain, where the power may be provided by a power source. When the channel of the transistor is fabricated from a conductive polymer, such as PEDOT:PSS, the drain voltage may have a bias between -0.1 and -0.6V DC. It will be understood that the drain voltage may be biased differently depending on the sensor material and/or sensor application. Preferably, when the sensor is formed from PEDOT:PSS, the drain voltage may not exceed 0.6V when the implantable apparatus is implanted onto the brain, due to unwanted effects. It will be understood that this drain voltage is to serve merely as a non-limiting example. The drain voltage used may depend on a biologically safe limit for a specific type of tissue or application and may thus vary significantly depending on, for example, where the sensor is implanted.

Figure 3 is a schematic diagram of a process to manufacture the implantable sensor. The method of manufacturing the implantable sensor comprises: providing a polymer substrate; forming source, drain and gate electrodes and a channel of the transistor on a first surface of the polymer substrate; and providing the light source and the power source on a second surface of the polymer substrate. The channel of the transistor may be formed of a conductive organic polymer material.

More specifically, the method comprises providing a sacrificial substrate (step 1) which is eventually removed, and depositing a polymeric base material, i.e. the polymer substrate, onto the substrate (step 2). The polymer substrate may be formed of a flexible material. Photolithography or similar techniques may be used to pattern conductive/metal interconnects on the polymer substrate (step 3). A photoresist may be used during the patterning process of step 3, and this may be removed once the patterning is complete (i.e. after the conductive/metal interconnects have been deposited/patterned). Metal/conductive interconnects may be deposited onto the polymer substrate (step 3), and an insulative material is provided over the interconnects (step 4). Patterning or etching is used to form the location of the sensing channel of the transistor (step 5). The material which forms the sensing channel of the transistor is deposited (step 6). The interconnects and sensing channel are covered in an insulating material, such as a polymer (step 7). This polymer may be the same or a different polymer to that which forms the polymer substrate. The sensing channel is exposed by removing some of the insulating material (step 8). The intermediate product which has been produced so far is inverted (step 9). Communication connection channels are formed within the polymer substrate (step 10), and the light source and power source are provided over the communication connection channels (step 11), so that the sensing channel is effectively connected to the light source and power source. The intermediate product is then removed from the sacrificial substrate (step 12) to form the implantable sensor.

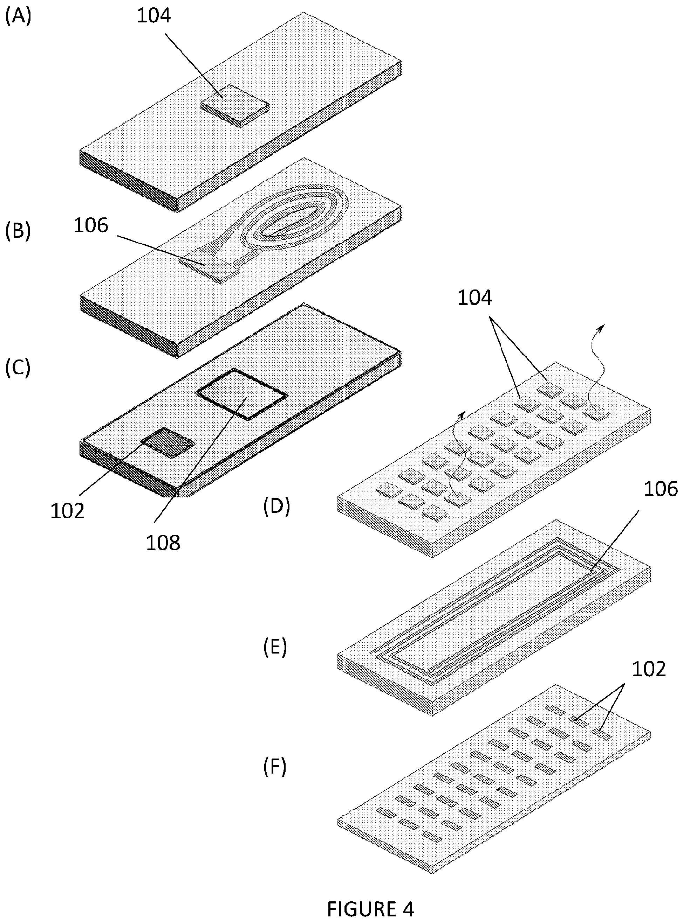

Figures 4A to 4C show diagrams of layers of the implantable sensor having a single light source, and Figures 4D to 4F show diagrams of layers of the implantable sensor having multiple light sources and multiple transistors. Figure 4A shows a single light source 104, Figure 4B shows a receiver and a power source, and Figure 4C shows a sensing channel of the transistor 102 and magnetic component 108. Figure 4D shows a plurality of light sources 104, Figure 4E shows a receiver and a power source, and Figure 4F shows sensing channels of a plurality of transistors 102. The light sources 104 in Figure 4D may be of the same type, or different.

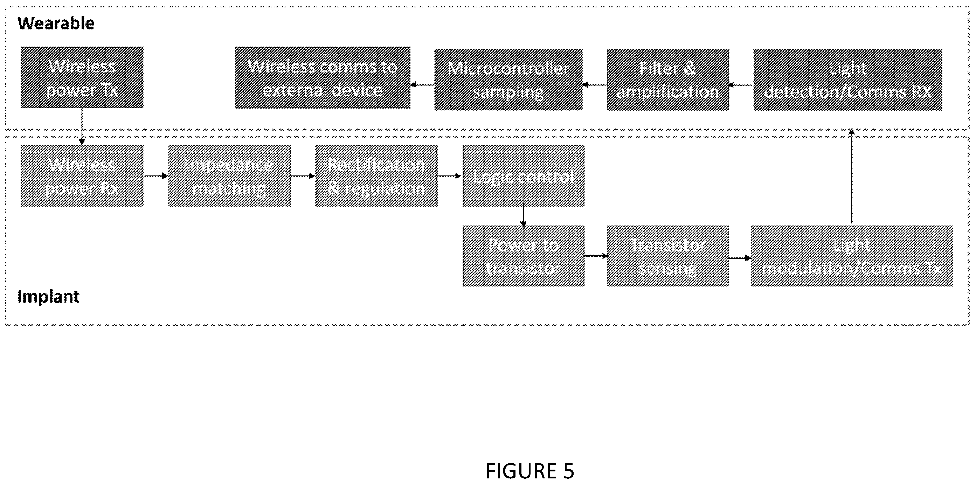

Figure 5 is a block diagram showing example processes performed by the system 10 of Figure 1. It will be understood that the system 10 may implement some or all of the processes shown here.

Figures 6A and 6B are schematic diagrams of an alternative process to manufacture the implantable sensor. The method of manufacturing the implantable sensor comprises: providing a polymer substrate; forming a channel of the at least one transistor on a first surface of the polymer substrate; forming source, drain and gate electrodes of the transistor on a second surface of the polymer substrate; providing the light source and the power source on the second surface of the polymer substrate. One way to form the channel may comprise forming a hole or aperture through the polymer substrate, and providing the channel material in or over this hole such that the channel material is accessible on both the first and second surface. The electrodes may then be provided on one of the surfaces (e.g. the second surface).

In cases where the sensor comprises a plurality of transistors, the method may further comprise forming a channel of at least one transistor on the first surface of the polymer substrate, and a channel of at least one other transistor is provided on the second surface of the polymer substrate.

The method may further comprise: providing an insulating layer on the source, drain and/or gate electrodes of the at least one transistor; forming at least two exposed terminals in the insulating layer; and placing the light source in contact with the at least two exposed terminals. The at least one channel of the transistor may be formed of a conductive organic polymer material. The exposed terminals may electrically connect the light source to the transistor.

More specifically, the method comprises providing a sacrificial substrate (step 1) which is eventually removed, and depositing an anti-adhesive layer (step 2) onto the substrate. The anti-adhesive layer prevents adhesion between the sacrificial substrate and any materials that are further deposited onto the sacrificial substrate and the anti-adhesive layer. Next, polymeric base material, i.e. the polymer substrate, is deposited onto the sacrificial substrate (step 3) which is coated with the anti-adhesive. The polymer substrate may be formed of a flexible material. Patterning by photolithography and/or etching may be used to form the location of the sensing channel of the at least one transistor (step 4). Any other suitable method may be used to form the location of and/or sensing channel of the transistor. Next, photolithography or similar techniques may be used to pattern conductive/metal interconnects on the polymer substrate (step 5). Alternatively, patterning of conductive/metal interconnects may take place after the channel is formed (step 6 below). That is, steps 5 and 6 may be interchangeable in order. A photoresist may be used during the patterning process of step 5, and this may be removed once the patterning is complete (i.e., after the conductive/metal interconnects have been deposited/patterned). Metal/conductive interconnects may be deposited onto the polymer substrate (step 5). The material which forms the at least one sensing channel is deposited and patterned next (step 6). Photolithography or similar techniques may be used to pattern the channel-forming material. A photoresist may be used during the patterning process of step 6, and this may be removed once the patterning is complete (i.e., after the channel-forming material have been deposited/patterned). Figure 6a shows one sensing channel being formed, with the sensing channel located on one side of the sensor. In the example shown in Figure 6a, the sensing channel is located towards the bottom of the sensor, i.e. the side of the sensor that is shown to be in contact with the anti-adhesive layer. Figure 6b shows two sensing channels being formed, with one sensing channel located on the side of the sensor shown to be in contact with the anti-adhesive layer, and the other sensing channel shown to be on the opposite side of the sensor.

The conductive interconnects (i.e. gate, drain and/or source electrodes) may be covered in an insulating material such as a polymer (step 7). This polymer may be the same or a different polymer to that which forms the polymer substrate. The insulating material may, for example be Parylene-C (PaC). The insulating material may, for example, be vapour deposited onto the conducting material. Directly vapour depositing the insulating material onto the conducting material may improve bonding between both layers of material and may therefore be advantageous. Connections for communication are formed on the insulating material (step 8), for example, using patterning techniques. Additionally, locations for a subset of sensing channels are also formed in this step, for example, using patterning techniques. That is, optionally, more than one sensing channel may be formed, as shown in Figure 6b. If two or more sensing channels are formed, they may be formed such that at least one of the sensing channels is exposed on one side of the sensor and at least one other sensing channel is exposed on the other side of the sensor. This is shown in Figure 6b, which shows two sensing channels. In this way, physiological signals from at least two sides of the sensor may be collected. The light source and power source are provided over the communication channels (step 9), so that the sensing channel is effectively connected to the light source and power source. The intermediate product is then removed from the sacrificial substrate (step 10) to form the implantable sensor. The anti-adhesive layer may facilitate removal of the sacrificial substrate at this point. In this alternative manufacturing approach, all components of the sensor are placed on one surface of the substrate only. This means that there is no need to turn over the substrate during the manufacturing process to achieve placement of a sensing channel on a different surface of the substrate as the light source, instead deposition of all layers can happen automatically and without an additional step of turning over the substrate.

Optionally, before the sensor is removed from the sacrificial substrate, any moisture sensitive parts of the sensor may be encapsulated to protect these parts. Moisture sensitive parts of the sensor may, for example, include an optical communication interface (i.e. an LED, OLED or any other light source/receiver). Encapsulating the light source/interface protects the light source/optical interface from contamination and/or failure. The optical interface may, for example, be protected by adding several layers of Parylene-C (PaC) onto the interface. The PaC layers may encapsulate at least the optical interface, and preferably the whole sensor apart from the at least one channel which records the physiological signal may be encapsulated in PaC. Adding a plurality of layers of PaC ensures that it is more difficult for moisture or other contaminants to penetrate the encapsulating layers.

Additionally, or alternatively, other encapsulation methods may be used. For example, the sensor may be encapsulated using layers of a polymer and a transparent metal oxide, such as, for example, aluminium oxide. The layers may alternate between the polymer and the metal oxide or there may be several layers of one material followed by at least one layer of the other material. That is, there may be one or more laminate layers, for example, at least one layer of a polymer, at least one layer of a metal oxide and further at least one layer of the polymer. The polymer may, for example, be PaC. Combining a polymer and a metal oxide may be advantageous as this may provide improved hermeticity, i.e. using more than one layering material may improve the properties of the encapsulation. Other encapsulation materials such as glass, rubber (e.g. silicone rubber) or any other suitable low temperature hermetic sealing method may be used.

Alternatively, the sensor may be encapsulated in a ceramic shell that comprises at least one window. The window(s) may be formed of a material such as, for example, glass or ruby, which provides the required hermetic properties, while ensuring excellent optical properties. That is, it is important that the encapsulation material does not compromise transmitting of light from the light source, and thus, any encapsulation material needs to be sufficiently optically transparent. Optically transparent may mean optically transparent at a predetermined target wavelength or frequency at which the sensor's light source and the detector are operating. Optically transparent may also mean optically transparent at one or more target wavelengths or target frequencies. For example, if a sensor's one or more light sources operate at two electromagnetic frequencies, the encapsulating material, through which light from the sensor's light source has to pass, would be optically transparent for at least the two electromagnetic frequencies at which the sensor's one or more light sources are emitting light. Optically transparent may further mean that a substantial portion of light at a certain frequency or wavelength is not absorbed by the encapsulating material at a certain thickness used in the encapsulation of the sensor. For example, the encapsulation may have a thickness anywhere between around 1 *µm* and several *mm.* For example, this may mean that at least 90% of light emitted by the sensor's light source at a target frequency can still pass through the encapsulating material. It will however be appreciated that this number is a non-limiting example and the amount of light that needs to be able to pass through the encapsulating material may depend on a number of factors, such as how deep the sensor is implemented into the body and hence how much light is absorbed by tissue before the light reaches the detector.

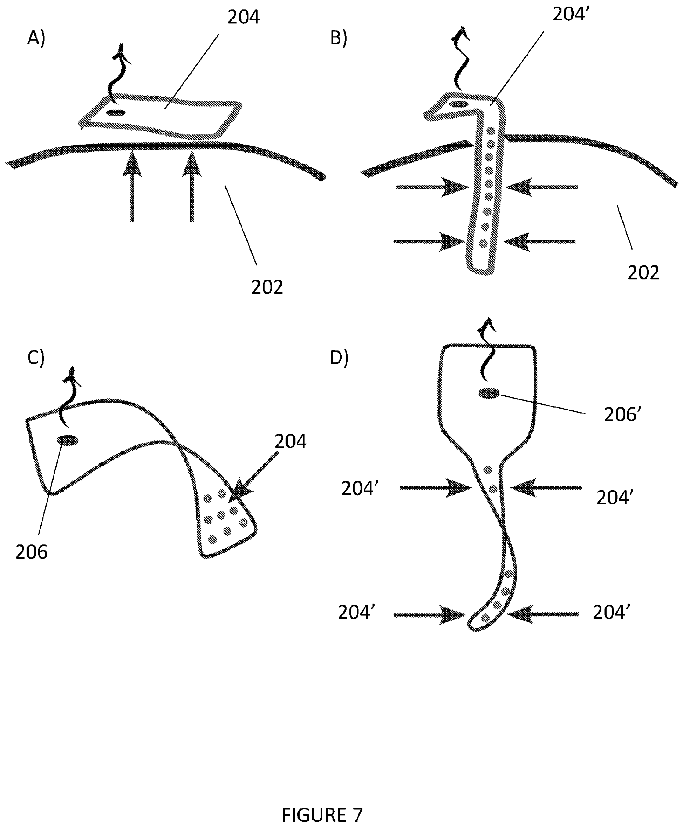

Figures 7B to 7D shows a number of alternative shapes the sensor 204 may take. The sensor may comprise a fold, with the light source provided on a first side of the fold and the at least one transistor provided on a second side of the fold. The first and second side may be at a right angle. The folded sensor may be L-shaped.

For example, the shape of the sensor may be adapted depending on the monitoring location of the sensor. Figures 7A and 7C show a flat sensor 204 that can be implanted onto the brain, as illustrated in Figure 7A. In this configuration, the sensor 204 senses physiological signals on the surface of the brain. The sensed physiological signals may then be transmitted outside the skull using a light source 206. The sensor may be arranged such that the light source 206 points towards the skull. Alternatively, it may be advantageous to adapt the shape of the sensor 204' such that the channel of the sensor points "into" the brain, as shown in Figure 7B. This way, physiological signals originating deeper inside the brain 202, not just on the surface of the brain may be monitored. Advantageously, adapting the shape of the sensor such that the sensor comprises a fold, as shown in Figure 7B means that a light source 206' of the sensor 204' is still on the surface of the brain and points towards the skull and thus light can still penetrate through the skull. That is, adapting the sensor 204' to have a bent shape or an L-shape as shown in Figures 7A and 7D means that the light source may point towards the skull, while the sensing part of the sensor (i.e. the at least one channel) may be located deeper inside the brain 202.

In both configurations shown, there may be more than one sensing channels, as is shown in the manufacturing process of Figure 7. That is, sensing may happen on either a first or a second surface of the sensor. This can be especially advantageous if the sensor is pointing into the brain, as this then enables physiological signals from several directions in the brain to be monitored.

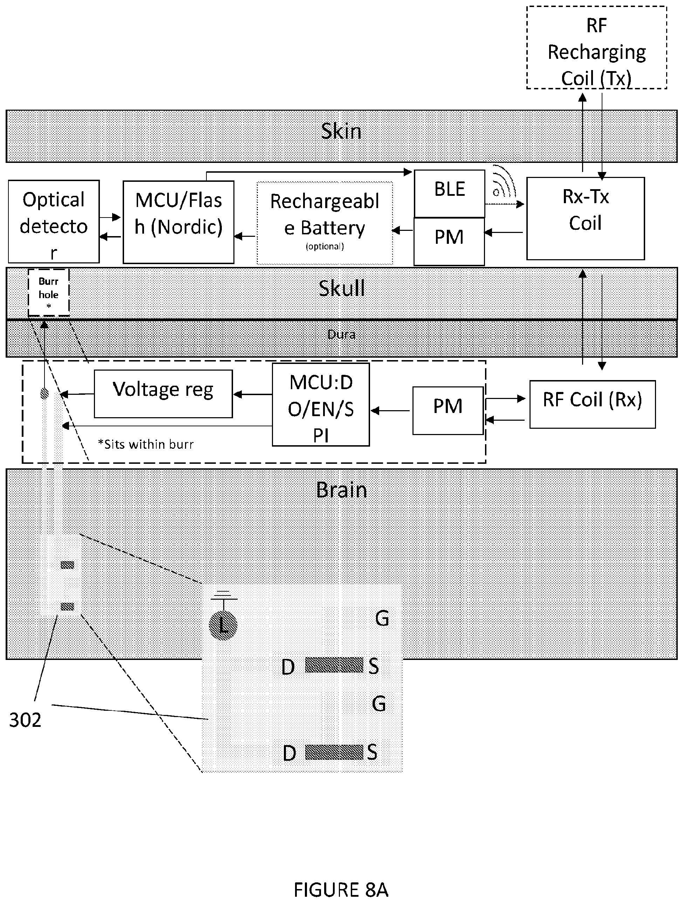

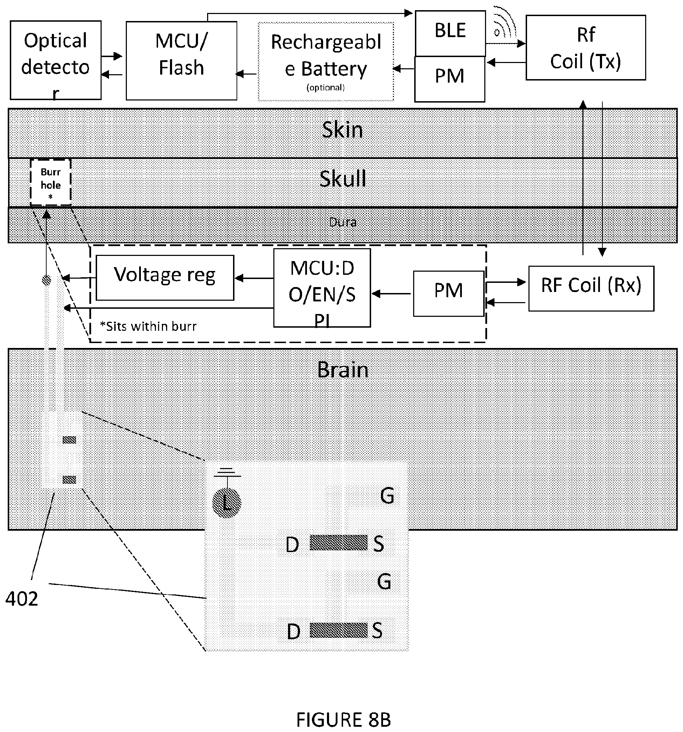

Figures 8A and 8B are block diagrams showing where example components of the system 10 of Figure 1 may be placed in relation to a user's anatomy. Figure 8A shows a fully implantable sensor and detector. The sensor 302 is implanted onto or into the brain. Merely for illustrative purposes, the sensor shown in Figure 8A includes two OECTs or IGTs with gate (G), source (S), and a drain (D) and channel (C). The OECTs or IGTs are modulating a light source (L). Other sensor configurations may include only one OECT or IGT or a plurality of OECTs and/or IGTs. To implant the sensor, a burr hole may be drilled into a user's skull to place the sensor and supporting electronics. For example, as shown in Figure 8A, a voltage regulator (Voltage reg), a microcontroller with digital output, encoding, and serial peripheral interface which may, for example, be used for transistor to light source switching (MCU:DO/EN/SPI), and power management (PM) may then be placed within the burr hole. A power source (RF Coil (Rx)) may be placed inside the skull and below the dura. When the power source is a wireless power source, the power source may be coupled to a power transmitter (Rx-Tx Coil) that transmits power to the wireless power source. The power transmitter may be part of the detector and may be placed outside the skull but under the skin. Further, the detector may comprise an optical detector, a microcontroller or other suitable processor with memory (MCU/Flash ), a wireless communication module, such as a Bluetooth^{®} Low Energy module (BLE), power management (PM), and optionally a rechargeable battery. In Figure 8A, the components of the detector are placed outside the skull but underneath the skin. Placing the detector underneath the skin may be more convenient for the user, as this removes the need to deal with a wearable device. If the power source is a wireless power source that is coupled to the power transmitter of the detector, there may be a further recharging power transmitter (RF Recharging Coil (Tx)) that may sit outside the skull and the skin and may be removable. The recharging power transmitter may be reversibly coupled to the power transmitter of the detector, which may then become a power receiver of the detector. The recharging power transmitter may transmit power to the detector for recharging a rechargeable battery of the detector. The power transmitter of the detector may thus be capable of transmitting power to the sensor and of receiving power from the recharging power transmitter.

The components shown and abbreviations used in Figure 8B have the same function and meaning as those described with reference to Figure 8A. Figure 8B shows a configuration of the sensor 402 and detector, where the sensor 402 is placed onto or in the brain, the supporting electronics, as described with reference to Figure 8A are placed inside a burr hole, and the detector is placed outside the skull and the skin. That is, the detector is not implanted but may be removably attached to a user's skin, for example, using an adhesive. Advantageously, in this configuration it is easier to replace the detector with an alternative detector, for example if the detector hardware or software is updated. Additionally, it is easier to make repairs to the detector. Further, this configuration means that no large surgical incision is needed to implant the detector. Additionally, when the detector comprises a battery, this may add further regulatory constraints. Thus, placing the detector outside the skull and skin may provide more flexibility in the detector design and any subsequent updates.

Although the RF coil (Rx) is shown to be under the dura in Figure 8B, the RF coil could also be provided under the skin. This may be useful for maximising power efficiency.

Those skilled in the art will appreciate that while the foregoing has described what is considered to be the best mode and where appropriate other modes of performing present techniques, the present techniques should not be limited to the specific configurations and methods disclosed in this description of the preferred embodiment. Those skilled in the art will recognise that present techniques have a broad range of applications, and that the embodiments may take a wide range of modifications without departing from any inventive concept as defined in the appended claims.

## Claims

1. An implantable sensor (100) for measuring a physiological signal, the sensor (100) comprising:
at least one transistor (102) for sensing the physiological signal;
at least one light source (104), coupled to the at least one transistor (102), for transmitting data to an external detector, wherein the at least one light source (104) emits light of a predetermined intensity and frequency in response to the sensed physiological signal;
at least one power source to power the at least one transistor (102) and the at least one light source (104); and
a magnetic component (108) for orienting the implantable sensor (100) within a body, such that light emitted by the at least one light source (104) is detectable by an external detector.

2. The sensor (100) as claimed in claim 1 wherein the sensor (100) comprises a plurality of transistors (102) coupled to a single light source (104).

3. The sensor (100) as claimed in claim 1 or 2 wherein the transistor (102) is an organic electrochemical transistor, OECT and/or an internally gated transistor, IGT; and/or
wherein the at least one power source is any of: an electromagnetic wireless power receiver, an acoustic power receiver, an optical power receiver, a wireless power receiver, an energy harvesting device, a battery, a supercapacitor, and a conductive polymer supercapacitor.

4. The sensor (100) as claimed in any of claims 1 to 3 wherein the sensor (100) comprises a polymer substrate, and source, drain and gate electrodes and a channel of the at least one transistor (102) are provided on a first surface of the polymer substrate;
preferably wherein the light source (104) and power source are provided on a second surface of the polymer substrate, and preferably wherein the at least one light source (104) and power source are coupled to the source and drain electrodes of the at least one transistor (102).

5. The sensor (100) as claimed in any of claims 1 to 3 wherein the sensor (100) comprises a polymer substrate, and a channel of the at least one transistor (102) is provided on a first surface of the polymer substrate, and source, drain and gate electrodes of the at least one transistor (102) are provided on a second surface of the polymer substrate;
preferably wherein the light source (104) and power source are provided on the second surface of the polymer substrate, and preferably wherein the at least one light source (104) and power source are coupled to the source and drain electrodes of the at least one transistor (102).

6. The sensor (100) as claimed in any of claims 1 to 3 wherein the sensor (100) comprises a polymer substrate and a plurality of transistors (102), and wherein a channel of at least one transistor of the plurality of transistors (102) is provided on a first surface of the polymer substrate and a channel of at least one other transistor of the plurality of transistors (102) is provided on a second surface of the polymer substrate;
preferably wherein the light source (104) and power source are provided on the second surface of the polymer substrate, and preferably wherein the at least one light source (104) and power source are coupled to the source and drain electrodes of the at least one transistor (102).

7. The sensor (100) as claimed in any of claims 4 to 6 wherein, for each transistor (102), the source and drain electrodes are insulated, and the channel is exposed; and/or
wherein, for each transistor (102), the gate electrode and channel is formed of a material suitable for sensing the physiological signal.

8. The sensor (100) as claimed in any of claims 4 to 7 wherein the substrate comprises a first portion and a second portion, wherein the first portion is at an angle relative to the second portion, and wherein the at least one light source (104) is on the first portion and the at least one transistor (102) is on the second portion.

9. The sensor (100) as claimed in any preceding claim wherein the at least one light source (104) is any one or more of: a light-emitting diode, an organic light-emitting diode, and a laser diode; and/or
wherein the sensor (100) further comprises components for delivering electrical stimulation.

10. The sensor (100) as claimed in any of claims 1 to 9 wherein the sensor (100) is partially encapsulated in an optically-transparent material, preferably wherein the optically-transparent material is a polymer and/or a metal oxide; or
wherein the sensor (100) is encapsulated in a protective material, and wherein the protective material comprises one or more windows or cut-outs.

11. The sensor (100) as claimed in any preceding claim, wherein the magnetic component (108) is adjacent to the at least one light source (104).

12. The sensor (100) as claimed in any preceding claim further comprising at least one modulator (110) for modulating light emitted by the at least one light source (104) in response to the sensed physiological signal;
preferably wherein the at least one modulator (110) modulates any one or more of the following properties of the emitted light: pulse, amplitude, frequency, phase and intensity, or wherein when no physiological signal is sensed by the transistor (102), the modulator (110) modulates light emitted by the light source so that light is emitted at a first frequency and a first intensity, and when the transistor (102) senses the physiological signal, the modulator (110) modulates light emitted by the light source so that light is emitted at a second frequency and a second intensity;

13. The sensor (100) as claimed in any one of claims 1 to 12 wherein the physiological signal sensed by the sensor (100) is a neural signal; or
wherein the physiological signal sensed by the sensor (100) is any one of: a chemical signal or substance, a biological signal or substance, or an electrical signal.

14. A wearable detector (200) for receiving light signals transmitted by an implantable sensor (100) as recited in any of claims 1 to 13, the detector comprising:
at least one photodetector (202) for detecting light signals transmitted by the implantable electronic sensor (100);
a processor (204) for processing the detected light signals;
a magnet (210) for magnetically coupling to the magnetic component of the implantable sensor (100) and orienting the implantable sensor (100) inside the body; and
a communication module (206) configured to transmit the detected light signals to an external computing device (300).

15. The detector (200) as claimed in claim 14 wherein the photodetector (202) is a near-infrared or visible light photodetector (202) and/or
wherein the detector further comprises a wireless power transfer module configured to wirelessly transfer power to the implantable sensor (100).

## Patentansprüche

1. Implantierbarer Sensor (100) zum Messen eines physiologischen Signals, wobei der Sensor (100) Folgendes umfasst:
mindestens einen Transistor (102) zum Erfassen des physiologischen Signals;
mindestens eine Lichtquelle (104), die mit dem mindestens einen Transistor (102) gekoppelt ist, zum Übertragen von Daten an einen externen Detektor, wobei die mindestens eine Lichtquelle (104) als Reaktion auf das erfasste physiologische Signal Licht mit einer vorbestimmten Intensität und Frequenz emittiert;
mindestens eine Leistungsquelle, um den mindestens einen Transistor (102) und die mindestens eine Lichtquelle (104) mit Leistung zu versorgen; und
eine magnetische Komponente (108) zum Ausrichten des implantierbaren Sensors (100) innerhalb eines Körpers, sodass Licht, das von der mindestens einen Lichtquelle (104) emittiert wird, durch einen externen Detektor detektierbar ist.

2. Sensor (100) nach Anspruch 1, wobei der Sensor (100) eine Vielzahl von Transistoren (102) umfasst, die mit einer einzigen Lichtquelle (104) gekoppelt sind.

3. Sensor (100) nach Anspruch 1 oder 2, wobei der Transistor (102) ein organischer elektrochemischer Transistor, OECT, und/oder ein intern gesteuerter Transistor, IGT, ist; und/oder
wobei die mindestens eine Leistungsquelle eines von Folgendem ist: ein elektromagnetischer drahtloser Leistungsempfänger, ein akustischer Leistungsempfänger, ein optischer Leistungsempfänger, ein drahtloser Leistungsempfänger, eine Energiegewinnungsvorrichtung, eine Batterie, ein Superkondensator und ein leitfähiger Polymer-Superkondensator.

4. Sensor (100) nach einem der Ansprüche 1 bis 3, wobei der Sensor (100) ein Polymersubstrat umfasst und Source-, Drain- und Gate-Elektroden und ein Kanal des mindestens einen Transistors (102) auf einer ersten Oberfläche des Polymersubstrats bereitgestellt sind;
wobei vorzugsweise die Lichtquelle (104) und die Leistungsquelle auf einer zweiten Oberfläche des Polymersubstrats bereitgestellt sind, und wobei vorzugsweise die mindestens eine Lichtquelle (104) und die Leistungsquelle mit den Source- und Drain-Elektroden des mindestens einen Transistors (102) gekoppelt sind.

5. Sensor (100) nach einem der Ansprüche 1 bis 3, wobei der Sensor (100) ein Polymersubstrat umfasst und ein Kanal des mindestens einen Transistors (102) auf einer ersten Oberfläche des Polymersubstrats bereitgestellt ist und Source-, Drain- und Gate-Elektroden des mindestens einen Transistors (102) auf einer zweiten Oberfläche des Polymersubstrats bereitgestellt sind;
wobei vorzugsweise die Lichtquelle (104) und die Leistungsquelle auf der zweiten Oberfläche des Polymersubstrats bereitgestellt sind, und wobei vorzugsweise die mindestens eine Lichtquelle (104) und die Leistungsquelle mit den Source- und Drain-Elektroden des mindestens einen Transistors (102) gekoppelt sind.

6. Sensor (100) nach einem der Ansprüche 1 bis 3, wobei der Sensor (100) ein Polymersubstrat und eine Vielzahl von Transistoren (102) umfasst, und wobei ein Kanal mindestens eines Transistors der Vielzahl von Transistoren (102) auf einer ersten Oberfläche des Polymersubstrats bereitgestellt ist und ein Kanal mindestens eines anderen Transistors der Vielzahl von Transistoren (102) auf einer zweiten Oberfläche des Polymersubstrats bereitgestellt ist;
wobei vorzugsweise die Lichtquelle (104) und die Leistungsquelle auf der zweiten Oberfläche des Polymersubstrats bereitgestellt sind, und wobei vorzugsweise die mindestens eine Lichtquelle (104) und die Leistungsquelle mit den Source- und Drain-Elektroden des mindestens einen Transistors (102) gekoppelt sind.

7. Sensor (100) nach einem der Ansprüche 4 bis 6, wobei für jeden Transistor (102) die Source- und Drain-Elektroden isoliert sind und der Kanal freigelegt ist; und/oder
wobei für jeden Transistor (102) die Gate-Elektrode und der Kanal aus einem Material gebildet sind, das zum Erfassen des physiologischen Signals geeignet ist.

8. Sensor (100) nach einem der Ansprüche 4 bis 7, wobei das Substrat einen ersten Teil und einen zweiten Teil umfasst, wobei der erste Teil in einem Winkel relativ zu dem zweiten Teil liegt und wobei sich die mindestens eine Lichtquelle (104) auf dem ersten Teil befindet und sich der mindestens eine Transistor (102) auf dem zweiten Teil befindet.

9. Sensor (100) nach einem der vorhergehenden Ansprüche, wobei die mindestens eine Lichtquelle (104) eines oder mehrere von Folgendem ist: eine Leuchtdiode, eine organische Leuchtdiode und eine Laserdiode; und/oder wobei der Sensor (100) ferner Komponenten zum Abgeben einer elektrischen Stimulation umfasst.

10. Sensor (100) nach einem der Ansprüche 1 bis 9, wobei der Sensor (100) teilweise in einem optisch transparenten Material eingekapselt ist, wobei das optisch transparente Material vorzugsweise ein Polymer und/oder ein Metalloxid ist; oder
wobei der Sensor (100) in einem Schutzmaterial eingekapselt ist, und wobei das Schutzmaterial ein oder mehrere Fenster oder einen oder mehrere Ausschnitte umfasst.

11. Sensor (100) nach einem der vorhergehenden Ansprüche, wobei die magnetische Komponente (108) an die mindestens eine Lichtquelle (104) angrenzt.

12. Sensor (100) nach einem der vorhergehenden Ansprüche, ferner umfassend mindestens einen Modulator (110) zum Modulieren von Licht, das von der mindestens einen Lichtquelle (104) als Reaktion auf das erfasste physiologische Signal emittiert wird;
wobei vorzugsweise der mindestens eine Modulator (110) eine oder mehrere der folgenden Eigenschaften des emittierten Lichts moduliert: Impuls, Amplitude, Frequenz, Phase und Intensität, oder wobei, wenn kein physiologisches Signal durch den Transistor (102) erfasst wird, der Modulator (110) Licht moduliert, das durch die Lichtquelle emittiert wird, sodass Licht mit einer ersten Frequenz und einer ersten Intensität emittiert wird, und wenn der Transistor (102) das physiologische Signal erfasst, der Modulator (110) Licht moduliert, das durch die Lichtquelle emittiert wird, sodass Licht mit einer zweiten Frequenz und einer zweiten Intensität emittiert wird.

13. Sensor (100) nach einem der Ansprüche 1 bis 12, wobei das physiologische Signal, das durch den Sensor (100) erfasst wird, ein neuronales Signal ist; oder
wobei das physiologische Signal, das durch den Sensor (100) erfasst wird, eines von Folgendem ist: ein chemisches Signal oder eine chemische Substanz, ein biologisches Signal oder eine biologische Substanz oder ein elektrisches Signal.

14. Tragbarer Detektor (200) zum Empfangen von Lichtsignalen, die durch einen implantierbaren Sensor (100) nach einem der Ansprüche 1 bis 13 übertragen werden, wobei der Detektor Folgendes umfasst:
mindestens einen Photodetektor (202) zum Detektieren von Lichtsignalen, die durch den implantierbaren elektronischen Sensor (100) übertragen werden;
einen Prozessor (204) zum Verarbeiten der detektierten Lichtsignale;
einen Magneten (210) zum magnetischen Koppeln mit der magnetischen Komponente des implantierbaren Sensors (100) und Ausrichten des implantierbaren Sensors (100) innerhalb des Körpers; und
ein Kommunikationsmodul (206), das dazu ausgelegt ist, die detektierten Lichtsignale an eine externe Rechenvorrichtung (300) zu übertragen.

15. Detektor (200) nach Anspruch 14, wobei der Photodetektor (202) ein Photodetektor (202) für Nahinfrarot oder sichtbares Licht ist und/oder
wobei der Detektor ferner ein drahtloses Leistungsübertragungsmodul umfasst, das dazu ausgelegt ist, Leistung drahtlos an den implantierbaren Sensor (100) zu übertragen.

## Revendications

1. Capteur implantable (100) destiné à mesurer un signal physiologique, le capteur (100) comprenant :
au moins un transistor (102) pour capter le signal physiologique ;
au moins une source lumineuse (104), couplée à l'au moins un transistor (102), destinée à transmettre des données à un détecteur externe, l'au moins une source lumineuse (104) émettant de la lumière d'intensité et de fréquence prédéterminées en réponse au signal physiologique capté ;
au moins une source de puissance pour alimenter l'au moins un transistor (102) et l'au moins une source lumineuse (104) ; et
un composant magnétique (108) destiné à orienter le capteur implantable (100) à l'intérieur d'un corps, de sorte que la lumière émise par l'au moins une source lumineuse (104) soit détectable par un détecteur externe.

2. Capteur (100) selon la revendication 1, le capteur (100) comprenant une pluralité de transistors (102) couplés à une seule source lumineuse (104).

3. Capteur (100) selon la revendication 1 ou 2, dans lequel le transistor (102) est un transistor électrochimique organique, OECT, et/ou un transistor à grille interne, IGT ; et/ou
dans lequel l'au moins une source de puissance est l'un quelconque des éléments suivants : un récepteur de puissance sans fil électromagnétique, un récepteur de puissance acoustique, un récepteur de puissance optique, un récepteur de puissance sans fil, un dispositif de récupération d'énergie, une batterie, un supercondensateur, et un supercondensateur polymère conducteur.

4. Capteur (100) selon l'une quelconque des revendications 1 à 3, le capteur (100) comprenant un substrat polymère, et des électrodes de source, de drain et de grille et un canal de l'au moins un transistor (102) étant disposés sur une première surface du substrat polymère ;
de préférence, dans lequel la source lumineuse (104) et la source de puissance sont disposées sur une deuxième surface du substrat polymère et, de préférence, dans lequel l'au moins une source lumineuse (104) et l'au moins une source de puissance sont couplées aux électrodes de source et de drain de l'au moins un transistor (102).

5. Capteur (100) selon l'une quelconque des revendications 1 à 3, le capteur (100) comprenant un substrat polymère, et un canal de l'au moins un transistor (102) étant disposé sur une première surface du substrat polymère, et des électrodes de source, de drain et de grille de l'au moins un transistor (102) étant disposées sur une deuxième surface du substrat polymère ;
de préférence, dans lequel la source lumineuse (104) et la source de puissance sont disposées sur la deuxième surface du substrat polymère et, de préférence, dans lequel l'au moins une source lumineuse (104) et l'au moins une source de puissance sont couplées aux électrodes de source et de drain de l'au moins un transistor (102).

6. Capteur (100) selon l'une quelconque des revendications 1 à 3, le capteur (100) comprenant un substrat polymère et une pluralité de transistors (102), et un canal d'au moins un transistor de la pluralité de transistors (102) étant disposé sur une première surface du substrat polymère et un canal d'au moins un autre transistor de la pluralité de transistors (102) étant disposé sur une deuxième surface du substrat polymère ;
de préférence, dans lequel la source lumineuse (104) et la source de puissance sont disposées sur la deuxième surface du substrat polymère et, de préférence, dans lequel l'au moins une source lumineuse (104) et l'au moins une source de puissance sont couplées aux électrodes de source et de drain de l'au moins un transistor (102).

7. Capteur (100) selon l'une quelconque des revendications 4 à 6 dans lequel, pour chaque transistor (102), les électrodes de source et de drain sont isolées, et le canal est à découvert ; et/ou
dans lequel, pour chaque transistor (102), l'électrode de grille et le canal sont formés d'un matériau adapté à capter le signal physiologique.

8. Capteur (100) selon l'une quelconque des revendications 4 à 7, dans lequel le substrat comprend une première partie et une deuxième partie, dans lequel la première partie forme un angle par rapport à la deuxième partie, et dans lequel l'au moins une source lumineuse (104) se trouve sur la première partie et l'au moins un transistor (102) se trouve sur la deuxième partie.

9. Capteur (100) selon l'une quelconque des revendications précédentes, dans lequel l'au moins une source lumineuse (104) est l'un quelconque ou plusieurs des éléments suivants : une diode électroluminescente, une diode électroluminescente organique, et une diode laser ; et/ou
le capteur (100) comprenant en outre des composants destinés à délivrer une stimulation électrique.

10. Capteur (100) selon l'une des revendications 1 à 9, le capteur (100) étant partiellement encapsulé dans un matériau optiquement transparent, de préférence le matériau optiquement transparent étant un polymère et/ou un oxyde métallique ; ou
le capteur (100) étant encapsulé dans un matériau de protection, et le matériau de protection comprenant une ou plusieurs fenêtres ou découpes.

11. Capteur (100) selon l'une quelconque des revendications précédentes, dans lequel le composant magnétique (108) est adjacent à l'au moins une source lumineuse (104).

12. Capteur (100) selon l'une quelconque des revendications précédentes, comprenant en outre au moins un modulateur (110) destiné à moduler la lumière émise par l'au moins une source lumineuse (104) en réponse au signal physiologique capté ;
de préférence, dans lequel l'au moins un modulateur (110) module l'une quelconque ou plusieurs des propriétés suivantes de la lumière émise : impulsion, amplitude, fréquence, phase et intensité, ou dans lequel, lorsqu'aucun signal physiologique n'est capté par le transistor (102), le modulateur (110) module la lumière émise par la source lumineuse de sorte que la lumière soit émise à une première fréquence et à une première intensité et, lorsque le transistor (102) capte le signal physiologique, le modulateur (110) module la lumière émise par la source lumineuse de sorte que la lumière soit émise à une deuxième fréquence et à une deuxième intensité.

13. Capteur (100) selon l'une quelconque des revendications 1 à 12, dans lequel le signal physiologique capté par le capteur (100) est un signal neuronal ; ou
dans lequel le signal physiologique capté par le capteur (100) est l'un quelconque des éléments suivants : un signal ou une substance chimique, un signal ou une substance biologique, ou un signal électrique.

14. Détecteur porté sur soi (200) destiné à recevoir des signaux lumineux transmis par un capteur implantable (100) selon l'une quelconque des revendications 1 à 13, le détecteur comprenant :
au moins un photodétecteur (202) destiné à détecter des signaux lumineux transmis par le capteur implantable électronique (100) ;
un processeur (204) destiné à traiter les signaux lumineux détectés ;
un aimant (210) destiné à être couplé magnétiquement au composant magnétique du capteur implantable (100) et à orienter le capteur implantable (100) à l'intérieur du corps ; et
un module de communication (206) configuré pour transmettre les signaux lumineux détectés à un dispositif informatique externe (300).

15. Détecteur (200) selon la revendication 14, dans lequel le photodétecteur (202) est un photodétecteur de lumière proche infrarouge ou visible (202) et/ou
le détecteur comprenant en outre un module de transfert de puissance sans fil configuré pour transférer sans fil de la puissance au capteur implantable (100).
